## Europäisches Patentamt

(19) **European Patent Office**

**Office européen des brevets**

(11) Veröffentlichungsnummer: **0 135 657**

**B1**

(12) **EUROPÄISCHE PATENTSCHRIFT**

(45) Veröffentlichungstag der Patentschrift:
27.12.89

(21) Anmeldenummer: **84104129.6**

(22) Anmeldetag: **13.04.84**

(51) Int. Cl.⁴: **A 61 K 31/495,** A 61 K 31/505, A 23 K 1/16, A 23 K 1/17 // (A61K31/495, 31:47, 31:505, 31:16),(A61K31/505, 31:495, 31:47, 31:16)

(54) Arzneimittel und/oder Futtermittelkonzentrate mit antibakterieller Wirkung.

(30) Priorität: **15.04.83  HU 131883**

(43) Veröffentlichungstag der Anmeldung:
**03.04.85 Patentblatt 85/14**

(45) Bekanntmachung des Hinweises auf die Patenterteilung:
**27.12.89 Patentblatt 89/52**

(84) Benannte Vertragsstaaten:
**AT BE CH DE FR GB IT LI NL SE**

(56) Entgegenhaltungen:
CH-A-619 460
US-A-3 021 216
US-A-3 433 871

CHEMICAL ABSTRACTS, Band 99, Nr. 23, 5. Dezember 1983, Seite 463, Zusammenfassung Nr. 191571p, Columbus, Ohio, US; & HU - A - 25 432 (PHYLAXIA OLTOANYAG-ES TAPSZERTERMELO VALLALAT) 28-07-1983
J. AM. VET. MED. ASSOC., Nr. 6, 15. März 1979, Seiten 597-600; D.O. FARRINGTON et al.: "Effect of carbadox on growth, feed utilization, and development of nasal turbinate lesions in swine infected with Bordetella bronchiseptica"

Die Akte enthält technische Angaben, die nach dem Eingang der Anmeldung eingereicht wurden und die nicht in dieser Patentschrift enthalten sind.

(73) Patentinhaber: **EGIS GYOGYSZERGYAR, Kereszturi ut 30- 38, Budapest X (HU)**

(72) Erfinder: **Magyar, Károly, Attila u. 131, Budapest I (HU)**
Erfinder: **Kelemen, Jozsef, Dr., Tulipán u. 10, Budapest II (HU)**
Erfinder: **Benko, Pál, Dr., Tartsay V. u. 7, Budapest XII (HU)**
Erfinder: **Simon, Ferenc, Dr., Szakasits Arpád u. 64/b, Budapest XI (HU)**
Erfinder: **Romváry, Attila, Dr., Bimbo u. 141/a, Budapest II (HU)**
Erfinder: **Varga, János, Dr., Bokányi u. 88, Budapest XVIII (HU)**
Erfinder: **Eliás, Béla, Dr., Szent István park 20/a, Budapest XIII (HU)**
Erfinder: **Mándi, Attila, Dr., Endrödi S. u. 7/a, Budapest II (HU)**

(74) Vertreter: **Beszédes, Stephan G., Dr. Patentanwalt, Münchener Strasse 80a Postfach 1168, D-8060 Dachau (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Arzneimittel mit antibakterieller Wirkung, die besonders gut in der Veterinärmedizin anwendbar sind, und Futtermittelkonzentrate mit antibakterieller Wirkung.

In der Schweinezucht bedeutet die Schnupfenkrankheit von Schweinen sehr große Gesundheitsprobleme und ist mit großen wirtschaftlichen Schäden verbunden. Bei dieser Krankheit erfolgt die Atrophie der Knochensubstanz der Nasenmuschel durch die Infektion der Schweine mit den Bakterien Bordatella bronchiseptica. Die Schnupfenkrankheit ist weitverbreitet und etwa 50 bis 60 % des Schweinebestandes ist mit Bordatella bronchiseptica infiziert. In der klinischen Form der Schnupfkrankheit tritt eine blutige eitrige Nasensekretion auf und die Nase wird schief. Obwohl diese schwere klinische Form der Krankheit lediglich 8 bis 10 % des Schweinebestandes vorkommt, verursacht auch die subklinische Form der Krankheit wesentliche wirtschaftliche Schäden, weil die Tiere sich langsamer vergrößern und sowohl die Futtermittelverwertung als auch die Gewichtszunahme um etwa 10 % vermindert wird.

Die mit Bordatella bronchiseptica infizierten Schweine werden nach der jetzigen Praxis meistens mit Antibiotica, Sulfonamiden oder durch 2,4-Diamino-5-[3',4',5'-tri-(methoxy)-benzyl]-pyrimidin {Trimethoprim} potenzierten Sulfonamiden behandelt. Die Bakterien wurden aber gegen diese Wirkstoffe resistent, weswegen ihre Wirksamkeit ungenügend ist (Vet. Record, 106 [1980] {22}, 462 bis 463).

Die mit Bordatella bronchiseptica infizierten Tiere können auch mit 2-[(Methoxycarbonylhydrazono)-methylen]-chinoxalin-1,4-dioxyd {Carbadox®} behandelt werden und auf diese Weise werden verhältnismäßig günstige Ergebnisse erreicht (J. Am. Vet. Med. Assoc., 6 [1979], 597 bis 600). Zur wirksamen Behandlung werden aber verhältnismäßig hohe Dosen benötigt.

Es besteht somit ein Bedarf an Arzneimitteln und/oder Futtermittelkonzentraten, die sowohl zur Prophylaxe als auch zur Therapie von bakteriellen Infektionen, besonders der Schnupfenkrankheit, vor allem bei Schweinen, in verhältnismäßig niedrigen Dosen geeignet sind.

Der Erfindung liegt daher die Aufgabe zugrunde, Arzneimittel und/oder Futtermittelkonzentrate mit antibakterieller Wirkung, welche niedrigeren Wirkstoffdosen als die nach dem Stand der Technik gut wirksam sind, zu schaffen.

Das Obige wurde überrasehenderweise durch die Erfindung erreicht.

Gegenstand der Erfindung sind Arzneimittel und/oder Futtermittelkonzentrate mit antibakterieller Wirkung mit einem Gehalt an

a) 2-[(methoxycarbonylhydrazono)-methylen]-chinoxalin-1,4-dioxyd {Carbadox®},

welche dadurch gekennzeichnet sind, daß sie als weitere[n] Wirkstoff(e)

b) 2-(Methyl)-5,7-di-(chlor)-8-(hydroxy)-chinolin {Chlorchinaldol}

enthalten.

Die CH-A-619 460 beschreibt Chinoxalin-di-N-oxid-Derivate, die strukturmäßig von dem erfindungsgemäß eingesetzten Chlorchinaldol verschieden sind, wobei von diesen bekannten Derivaten angegeben wird, daß sie das Wachstum von Haus- und Nutztieren fördernde Eigenschaften besitzen. Einen Hinweis, daß Chlorchinaldol in Kombination mit Carbadox zur Bekämpfung von Bordatella bronchiseptica von Tieren, insbesondere Schweinen, eine synergistische Wirkung besitzt, ist dieser Literaturstelle nicht zu entnehmen.

Die US-A-3 433 871 beschreibt Schiff'sche Basen, die auf 2-Formyl-chinoxalin-1,4-dioxide zurückgehen. Das erfindungsgemäß eingesetzte Chlorchinaldol fällt nicht unter diese bekannte Verbindungsgruppe. Von den unter diese Verbindungsgruppe fallenden Verbindungen wird angegeben, daß sie das Wachstum von Tieren begünstigen, chronische Atmungskrankheiten von Geflügeln bekämpfen, für eine Behandlung einer infektiösen Sinusitis in Truthähnen und im Urintrakt sowie zur Behandlung von systemischen und nichtsystemischen Infektionen in Tieren geeignet sind.

Die US-A-3 021 216 beschreibt Futterzusätze für Tiere, die zwar das erfindungsgemäß eingesetzte Chlorchinaldol umfassen können, es findet sich jedoch kein Hinweis darauf, daß das Chlorchinaldol in Kombination mit Carbadox eine synergistische Wirkung zur Bekämpfung von Bordatella bronchiseptica ausübt.

Nach einer vorteilhaften Ausführungsform enthalten die erfindungsgemäßen Arzneimittel und/oder Futtermittelkonzentrate zusätzlich

c) 4-Dimethylamino-3,5,6,10,12,12a-hexahydroxy-6-methyl-1,11-dioxo-1,4,4a,5,5a,6,11,12a-octahydronaphthacen-2-carbonsäureamid {Oxytetracyclin}.

Nach einer weiteren vorteilhaften Ausführungsform enthalten die erfindungsgemäßen Arzneimittel und/oder Futtermittelkonzentrate zusätzlich

d) 2,4-Diamino-5-[3',4',5'-tri-(methoxy)-benzyl]-pyrimidin {Trimethoprim}.

Dabei können nur das erstere [c)] oder nur das letztere [d)] oder beide vorliegen.

Nach einer zweckmäßigen Ausführungsform enthalten die erfindungsgemäßen Arzneimittel und/oder Futtermittelkonzentrate 1 oder mehr in Arzneimitteln und/oder Futtermittelkonzentraten übliche[s] feste[s] und/oder flüssige[s] Konfektionierungsmittel.

Nacheiner speziellen vorteilhaften Ausführungsform enthalten die erfindungsgemäßen Arzneimittel und/oder Futtermittelkonzentrate zusätzlich 1 oder mehr Salicylsäurederivat(e).

Nach einer zweckmäßigen Ausführungsform liegen die erfindungsgemäßen Arzneimittel und/oder Futtermittelkonzentrate in räumlich getrennter Aufteilung der Wirkstoffe in ein und derselben Verpackungs- beziehungsweise Applikationseinheit vor.

Es ist bevorzugt, daß in den erfindungsgemäßen Arzneimitteln und/oder Futtermittelkonzentraten das Gewichtsverhältnis von a) : b) : sofern vorhanden c) sofern vorhanden d) 1 bis 5 : 0,5 bis 100 : 0,1 bis 100 : 0,6 bis 100, insbesondere 1 : 1 bis 20 : sofern vorhanden 0,1 bis 20 : sofern vorhanden 0,6 bis 20, ganz besonders 1 : 1 bis 10 : sofern vorhanden 0,1 bis 10 : sofern vorhanden 0,6 bis 8, ist.

Vorzugsweise enthalten die erfindungsgemäßen Arzneimittel und/oder Futtermittelkonzentrate 1 bis 5 Gew.-Teil(e) 2-[(Methoxycarbonylhydrazono)-methylen]-chinoxalin-1,4-dioxyd {Carbadox®} <a)> und 0,5 bis 100 Gew.-Teil(e) 2-(Methyl)-5,7-di-(chlor)-8-(hydroxy)-chinolin {Chlorchinaldol} <b)> sowie gegebenenfalls 0,1 bis 100 Gew.-Teil(e) 4-Dimethylamino- 3,5,6,10,12,12a-hexahydroxy-6-methyl-1,11-dioxo-1,4,4a,5,5a,6,11,12a-octahydronaphthacen-2-carbonsäureamid {Oxytetracyclin} <c)> und/oder 0,6 bis 100 Gew.-Teil(e) 2,4-Diamino-5-[3',4',5'-tri-(methoxy)-benzyl]-pyrimidin {Trimethoprim} <d)>.

Die erfindungsgemäßen Arzneimittel und/oder Futtermittelkonzentrate können in erster Linie zur Prophylaxe und Therapie der Schnupfenkrankheit, insbesondere von Schweinen, verwendet werden.

Obwohl jeder der obigen Wirkstoffe eine antibakterielle Wirkung hat, sind verhältnismäßig hohe Dosen von 2-[Methoxycarbonylhydrazono)-methylen]-chinoxalin-1,4-dioxyd {Carbadox®} und 2,4-Diamino-5-[3',4',5'-tri-(methoxy)-benzyl] pyrimidin {Trimethoprim} bei alleiniger Verwendung derselben erforderlich. Was 2-(Methyl)-5,7-di-(chlor)-8-(hydroxy)-chinolin {Chlorchinaldol} betrifft, ist dieses eine bekannte Verbindung mit einer milden bactericiden und fungiciden Wirkung. Sie wird in erster Linie für dermatologische Zwecke, zur Behandlung von Hautinfektionen, verwendet. Obwohl bei 2-(Methyl)-5,7-di-(chlor)-8-(hydroxy)-chinolin {Chlorchinaldol} der in vitro bestimmte Wert der Mindesthemmkonzentration sehr niedrig ist, weshalb eine hohe Wirksamkeit zu erwarten wäre, ist es an sich zum Beispiel gegen Schnupfen unwirksam, wobei bei Untersuchungen in vivo die therapeutisch wirksame Dosis nicht erreicht wurde. 2-(Methyl)-5,7-di-(chlor)-8-(hydroxy)-chinolin {Chlorchinaldol} wird sehr schlecht absorbiert, weshalb im Blutplasma eine Konzentration von höchstens 5 µg/ml erreicht wird. Zur therapeutischen Wirkung wird jedoch eine wesentlich höhere Konzentration, nämlich etwa 15 bis 20 µ/ml, gebraucht. Mit 2-(Methyl)-5,7-di-(chlor)-8-(hydroxy)-chinolin {Chlorchinaldol} allein wird also die zur Therapie erforderliche Konzentration im Plasma nicht erreicht. In den erfindungsgemäßen Arzneimitteln und/oder Futtermittelkonzentraten ist die Rolle des 2-(Methyl)-5,7-di-(chlor)-8-(hydroxy)-chinolines {Chlorchinaldoles} eine ganz andere, und zwar verringert es überraschenderweise die Dosis der eigentlich therapeutisch wirksamen anderen Destandteile wesentlich, wie es aus der unten folgenden Tabelle hervorgeht. Es ist überraschend, daß die Mindesthemmkonzentrationen von 2-[(Methoxycarbonylhydrazono)-methylen]-chinoxalin-1,4-dioxyd {Carbadox®} und/oder 4-Dimethylamino-3,5,6,10,12,12a-hexahydroxy-6-methyl-1,11-dioxo-1,4,4a,5,5a,6,11,12a-octahydronaphthacen-2-carbonsäureamid {Oxytetracyclin} und/oder 2,4-Diamino-5-[3',4',5'-tri-(methoxy)-benzyl]-pyrimidin {Trimethoprim} gegen Bordatella bronchiseptica in Gegenwart einer sehr geringen Konzentration von 2-(Methyl)-5,7-di-(chlor)-8-(hydroxy)-chinolin {Chlorchinaldol} syneregistisch um 1 Größenordnung vermindert werden.

Diese Feststellung wird durch die folgenden Versuchsergebnisse bestätigt. Die Mindesthemmkoncentrationen von Kombinationen von 2 bis 4 der obigen Verbindungen in erfindungsgemäßen Arzneimitteln beziehungsweise der einzelnen obigen Verbindungen als vergleichssubstanzen wurden in vitro gegen die Bakterienstämme Bordatella bronchiseptica und Pasteurella multocid in einer Phenolrot und Glucose enthaltenden Brühe [Hersteller: Firma Difco] bestimmt. In der folgenden Tabelle sind die Werte der Mindesthemmkonzentration [MIC-Werte] in mg/l, die den Bakterienzuwachs 24. Stunden lang hemmen, zusammengestellt.

**Tabelle**

| Untersuchtes Material | Werte der Mindesthemmkonzentration [MIC-Werte] in mg/l | |
|---|---|---|
| Bezeichnung | in Bezug auf die Bakterienstämme | |
| | Bordatella bronchiseptica | Pasteurella multocida |
| 2-[(Methoxycarbonylhydrazono)-methylen]-chinoxalin-1,4-dioxyd {Carbadox®} | 50 | 10 |
| 2-(Methyl)-5,7-di-(chlor)-8-(hydroxy)-chinolin {Chlorchinaldol} | 0,5 | 0,5 |

3

| | | |
|---|---|---|
| 4-Dimethylamino-3,5,6,10,12,12a-hexahydroxy-6-methyl-1,11-dioxo-1,4,4a,5,5a,6,11,12a-octahydro-naphthacen-2-carbonsäureamid {Oxytetracyclin} | 5 | 5 |
| 2,4-Diamino-5-[3',4',5'-tri-(me-thoxy)-benzyl]-pyrimidin {Trimethoprim} | 50 | 50 |
| Erfindungsgemäßes Arzneimittelpräparat, bestehend aus: | | |
| 2-[(Methoxycarbonylhydrazono)-methylen]-chinoxalin-1,4-dioxyd {Carbadox®} <a)> | a) 5 + b) 0,5 | a) 5 + b) 0,5 |
| 2-(Methyl)-5,7-di-(chlor)-8-(hydroxy)-chinolin {Chlorchinaldol} <b)> | | |
| 2-[Methoxycarbonylhydrazono)-methylen]-chinoxalin-1,4-dioxyd {Carbadox®} <a)> | | |
| 2-(Methyl)-5,7-di-(chlor)-8-(hydroxy)-chinolin {Chlorchinaldol} <b)> | a) 5 + b) 0,5 + c) 0,5 | a) 5 + b) 0,5 + c) 0,5 |
| 4-Dimethylamino-3,5,6,10,12,12a-hexahydroxy-6-methyl-1,11-dioxo-1,4,4a,5,5a,6,11,12a-octahydro-naphthacen-2-carbonsäureamid {Oxytetracyclin} <c)> | | |
| 2-[(Methoxycarbonylhydrazono)-methylen]-chinoxalin-1,4-dioxyd {Carbadox®} <a)> | a) 5 + b) 0,5 + d) 5 | a) 5 + b) 0,5 + d) 5 |
| 2-(Methyl)-5,7-di-(chlor)-8-(hydroxy)-chinolin {Chlorchinaldol} <b)> | | |
| 2,4-Diamino-5-[3',4',5'-tri-(methoxy)-benzyl]-pyrimidin {Trimethoprim} <d)> | | |
| 2-[(Methoxycarbonylhydrazono)-methylen]-chinoxalin-1,4-dioxyd {Carbadox®} <a)> | a) 5 + b) 0,5 + c) 0,5 + d) 5 | a) 5 + b) 0,5 + c) 0,5 + d) 5 |
| 2-(Methyl)-5,7-di-(chlor)-8-(hydroxy)-chinolin {Chlorchinaldol} <b)> | | |

4

EP 0 135 657 B1

4-Dimethylamino-3,5,6,10,12,12a-
hexahydroxy-6-methyl-1,11-dioxo-
1,4,4a,5,5a,6,11,12a-octahydro-
naphthacen-2-carbonsäureamid
{Oxytetracyclin}
<c)>

2,4-Diamino-5-[3',4',5'-tri-(methoxy)-benzyl]-pyrimidin
{Trimethoprim}
<d)>

Die Werte der Mindesthemmkonzentration [MIC-Werte] bei den Kombinationen bedeuten, daß die angegebenen Wirkstoffe gemeinsam in den angegebenen Konzentrationen das Wachstum der beiden verschiedenen Bakterienstämme bereits hemmen.

Aus der obigen Tabelle geht hervor, daß eine Menge von nur 5 mg/l 2-[(Methoxycarbonylhydrazono)-methylen]-chinoxalin-1,4-dioxyd {Carbadox®} <a)> und 0,5 mg/l 4-Dimethylamino-3,5,6,10,12,12a-hexahydroxy-6-methyl-1,11-dioxo-1,4,4a,5,5a,6,11,12a-octahydronaphthacen-2-carbonsäureamid {Oxytetracyclin} <c)> und/oder 5 mg/l 2,4-Diamino-5-[3',4',5'-tri-(methoxy)-benzyl]-pyrimidin {Trimethoprim} <d)> in Gegenwart von 0,5 mg/l 2-(Methyl)-5,7-di-(chlor)-8-(hydroxy)-chinolin {Chlorchinaldol} <b)> zur Hemmung der geprüften Bakterien genügt.

In der obigen Tabelle ist die erreichte Wirkung immer die gleiche (Hemmung über 24 Stunden), nur die dazu notwendige Konzentration ist von Fall zu Fall verschieden. Der Synergismus der Wirkung in den erfindungsgemäßen Arzneimitteln und/oder Futtermittelkonzentraten zeigt sich darin, daß die Mindesthemmkonzentrationen der gegen die herangezogenen Bakterien wirksamen Substanzen 2-[(Methoxycarbonylhydrazono)-methylen]-chinoxalin-1,4-dioxyd {Carbadox®}, 4-Dimethylamino-3,5,6,10,12,12a-hexahydroxy-6-methyl-1,11-dioxo-1,4,4a,5,5a,6,11,12a-octahydronaphthacen-2-carbonsäureamid {Oxytetracyclin} und 2,4-Diamino-5-[3',4',5'-tri-(methoxy)-benzyl]-pyrimidin {Trimethoprim} durch das gegen diese praktisch unwirksame 2-(Methyl)-5,7-di-(chlor)-8-(hydroxy)-chinolin {Chlorchinaldol} in Kombination miteinander jeweils auf 1/10 verringert wird, wobei damit auch die rein additive Wirkung dieser Wirkstoffe bei weitem überschritten ist.

Ferner wurde festgestellt, daß die erfindungsgemäßen Arzneimittel und/oder Futtermittelkonzentrate auch das Wachstum von anderen Bakterienstämmen, wie Streptococcus, Erysipelothrix, Corynebacterium, Escherichia und Salmonella, hemmen. Daher können die erfindungsgemäßen Arzneimittel und/oder Futtermittelkonzentrate zur Behandlung auch anderer Infektionen als der Schnupfenkrankheit verwendet werden.

Nach einer vorteilhaften Ausführungsform enthalten die erfindungsgemäßen Arzneimittel und/oder Futtermittelkonzentrate 1 bis 5 Gew.-Teil(e) 2-[(Methoxycarbonylhydrazono)-methylen]-chinoxalin-1,4-dioxyd {Carbadox®} <a)> und 0,5 bis 100, insbesondere 1 bis 100, Gew.-Teil(e) 2-(Methyl)-5,7-di-(chlor)-8-(hydroxy)-chinolin {Chlorchinaldol} <b)>. Diese liefern günstige Ergebnisse bei der prophylaktischen und therapeutischen Behandlung der Schnupfenkrankheit von Schweinen. Nach einer bevorzugten Ausführungsform enthalten die erfindungsgemäßen Arzneimittel und/oder Futtermittelkonzentrate außer den vorstehend genannten Bestandteilen auch 0,1 bis 100, insbesondere 1 bis 100 Gew.-Teil(e) 4-Dimethylamino-3,5,6,10,12,12a-hexahydroxy-6-methyl-1,11-dioxo-1,4,4a,5,5a,6,11,12a-octahydronaphthacen-2-carbonsäureamid {Oxytetracyclin} <c)> und/oder 0,6 bis 100 Gew.-Teil(e) 2,4-Diamino-5-[3',4',5'-tri-(methoxy)-benzyl]-pyrimidin {Trimethoprim} <d)>. Diese sind besonders bei schweren Infektionen bevorzugt, weil die ausgezeichnete Absorption von 4-Dimethylamino-3,5,6,10,12,12a-hexahydroxy-6-methyl-1,11-dioxo-1,4,4a,5,5a,6,11,12a-octahydronaphthacen-2-carbonsäureamid {Oxytetracyclin} <c)> und 2,4-Diamino-5-[3',4',5'-tri-(methoxy)-benzyl]-pyrimidin {Trimethoprim} <d)> eine äußerst wirksame Therapie ermöglicht.

Als Konfektionierungsmittel können die erfindungsgemäßen Arzneimittel und/oder Futtermittelkonzentrate übliche feste und/oder flüssige Träger enthalten.

Die erfindungsgemäßen Arzneimittel können in Form von Arzneimittelpräparaten, die im allgemeinen in der Therapie und besonders in der Veterinärpraxis üblich sind, zum Beispiel als Tabletten, überzogene Tabletten, Pulvergemische, Injektionslösungen, Pillen, Boluse, Suspensionen beziehungsweise Emulsionen, vorliegen. Vorteilhafte Arzneimittelpräparate sind Pulvergemische, Boluse und Suspensionen.

Vorteilhafte Träger in den erfindungsgemäßen festen Arzneimittelpräparaten sind Glucose, Stärke, Siliciumdioxyd, 1 oder mehr Silikat(e), Sorbit, Milchzucker, Talk, Polyvinylpyrrolidon, Calciumphosphat und/oder Magnesiumstearat.

Vorteilhafte Träger in den erfindungsgemäßen flüssigen Arzneimittelpräparaten sind Wasser und/oder 1 oder mehr organische[s] Lösungsmittel, Emulgiermittel, Dispergiermittel, Suspendiermittel, Netzmittel, Antioxydationsmittel, Stabilisator(en) und/oder Süßungsmittel.

Vorteilhafte Träger in den erfindungsgemäßen Futtermittelkonzentraten sind Weizenmehl, Weisenkleie, Reiskleie (ölfrei), Maismehl, Sojamehl, Kaolin, Zeolithe, Calciumcarbonat und/oder Stärke und/oder 1 oder mehr Spurenelement(e), Vitamin(e) und/oder anorganische[s] Salz(e). Bevorzugte Träger sind die verschiedenen Stärken, beispielsweise Mais- und/oder Kartoffelstärke.

In den erfindungsgemäßen Arzneimittelpräparaten und/oder Futtermittelkonzentraten beträgt die Gesamtmenge der Wirkstoffe meistens 1 bis 90 Gew.-%, vorteilhaft 5 bis 20 Gew.-%.

Die erfindungsgemäßen Arzneimittelpräparate und/oder Futtermittelkonzentrate können in an sich bekannter Weise durch Vermischen der Wirkstoffe mit 1 oder mehr Konfektionierungsmittel(n) bereitet werden.

5

Falls es erwünscht ist, können die Gemische der Wirkstoffe und des beziehungsweise der Konfektionierungsmittel gemahlen werden. Die erfindungsgemäßen Arzneimittelpräparatsuspensionen können vor dem Gebrauch durch Suspendieren der erfindungsgemäßen Arzneimittelpräparatpulvergemische in Flüssigkeiten, wie Wasser oder Kamillentee, bereitet werden.

Die in den erfindungsgemäßen Arzneimitteln und/oder Futtermittelkonzentraten enthaltenen Wirkstoffe sind bekannte handelsübliche Verbindungen.

Im allgemeinen werden die erfindungsgemäßen Arzneimittel den Tieren peroral verabreicht.

Die erfindungsgemäßen Futtermittelkonzentrate können zum Bereiten von gebrauchsfertigen Tierfuttermitteln mit entsprechenden Futtermittelbestandteilen verdünnt und/oder mit entsprechenden Tierfuttermitteln vermischt werden. So erhaltene Futtermittel enthalten vorzugsweise 0,001 bis 0,005 Gew.-% (10 bis 50 ppm) 2-[(Methoxycarbonyl-hydrazono)-methylen]-chinoxalin-1,4-dioxyd {Carbadox®} <a)> und 0,0005 bis 0,1 Gew.-% (5 bis 1 000 ppm) 2-(Methyl)-5,7-di-(chlor)-8-(hydroxy)-chinolin {Chlorchinaldol} <b)> sowie gegebenenfalls 0,0001 bis 0,1 Gew.-% (1 bis 1 000 ppm) 4-Dimethylamino-3,5,6,10,12,12a-hexahydroxy-6-methyl-1,11-dioxo-1,4,4a,5,5a,6,11,12a-octahydro-naphthacen-2-carbonsäureamid {Oxytetracyclin} <c)> und/oder 0,0006 bis 0,1 Gew.-% (6 bis 1 000 ppm) 2,4-Diamini-5-[3',4',5'-tri-(methoxy)-benzyl]-pyrimidin {Trimethoprim} <d)>.

Zur Prophylaxe werden die Säue vorzugsweise 30 Tage lang behandelt. In diesem Fall wird die Behandlung zweckmäßig etwa 15 Tage vor den Wurf begonnen. Zweckmäßig werden dann die neugeborenen, 2 Wochen alten Ferkel auch 30 Tage lang behandelt.

Die Erfindung wurd an Hand der folgenden Beispiele näher erläutert.

**Beispiel 1**

Es wurden 1 kg 2-[(Methoxycarbonylhydrazono)-methylen]-chinoxalin-1,4-dioxyd {Carbadox®} <a)>, 10 kg 2-(Methyl)-5,7-di-(chlor)-8-(hydroxy)-chinolin {Chlorchinaldol} <b)>, 10 kg 4-Dimethylamino-3,5,6,10,12,12a-hexahydroxy-6-methyl-1,11-dioxo-1,4,4a,5,5a,6,11,12a-octahydronaphthacen-2-carbonsäureamid {Oxytetracyclin} <c)> und 79 kg Maisstärke miteinander vermischt und in einer Hammermühle vermahlen. Vor dem Gebrauch wurde 1 Gew.-Teil des erhaltenen Konzentrates mit 199 Gew.-Teilen eines üblichen Futtermittels der folgenden Zusammensetzung verdünnt:

| | |
|---|---|
| 20 Gew.-% | Mais, |
| 5 Gew.-% | Weizen, |
| 18 Gew.-% | Gerste, |
| 5 Gew.-% | Hafer, |
| 2 Gew.-% | Kleie, |
| 16 Gew.-% | extrahierte Soja, |
| 4 Gew.-% | Fischmehl, |
| 20 Gew.-% | Milchpulver, |
| 6 Gew.-% | Fettpulver und |
| 4 Gew.-% | einer aus Mineralsalzen, Vitaminen und Spurenelementen bestehenden Mischung. |

Die Schweine wurden mit dem erhaltenen Futtermittel gefüttert.

**Beispiel 2**

Es wurden 1 kg 2-[(Methoxycarbonylhydrazono)-methylen]-chinoxalin-1,4-dioxyd {Carbadox®} <a)> und 10 kg 2-(Methyl)-5,7-di-(chlor)-8-(hydroxy)-chinolin {Chlorchinaldol} <b)> mit 89 kg Kartoffelstärke vermischt und das Pulvergemisch wurde in einer Kugelmühle gemahlen. Aliquote des erhaltenen Pulvergemisches wurden vor dem Gebrauch in Kamillentee suspendiert. Die gut aufgerührte Suspension wurde Schweinen peroral verabreicht.

**Beispiel 3**

Es wurden 1 kg 2-[(Methoxycarbonylhydrazono)-methylen]-chinoxalin-1,4-dioxd {Carbadox®} <a)>, 8 kg 2-(Metyl)-5,7-di-(chlor)-8-(hydroxy)-chinolin {Chlorchinaldol} <b)>, 0,6 kg 2,4-Diamino-5-[3',4',5'-tri-(methoxy)-benzyl]-pyrimidin {Trimethoprim} <c)> und 90,4 kg Maisstärke miteinander vermischt. 1 Gew.-Teil des erhaltenen Konzentrates wurde vor dem Gebrauch mit 250 Gew.-Teilen eines üblichen Futtermittels der folgenden Zusammensetzung verdünnt:

| | |
|---|---|
| 38 Gew.-% | Mais, |
| 6 Gew.-% | Weizen, |
| 25 Gew.-% | Gerste, |
| 5 Gew.-% | Hafer, |
| 2,4 Gew.-% | Kleie, |
| 13 Gew.-% | extrahierte Soja, |
| 6 Gew.-% | Fischmehl, |

| 1,5 Gew.-% | Fettpulver, |
| 1 Gew.-% | Futterkalk, |
| 0,5 Gew.-% | Futtersalz und |
| 1,6 Gew.-% | einer aus Mineralsalzen Vitaminen und Spurenelementen bestehenden Mischung. |

**Beispiel 4**

Es wurden 5 kg 2-[(Methoxycarbonylhydrazono)-methylen]-chinoxalin-1,4-dioxyd {Carbadox®} <a)>, 25 kg 2-Methyl-5,7-di-(chlor)-8-(hydroxy)-chinolin {Chlorchinaldol} <b)>, 0,5 kg 4-Dimethylamino-3,5,6,10,12,12a-hexahydroxy-6-methyl-1,11-dioxo-1,4,4a,5,5a,6,11,12a-octahydronaphthacen-2-carbonsäureamid {Oxytetracyclin} <c)> und 40 kg 2,4-Diamino-5-[3',4',5'-tri(methoxy)benzyl]-pyrimidin {Trimethoprim} <d)> mit 29,5 kg Maismehl vermischt. 1 Gew.-Teil des gemahlenen Gemisches wurde vor dem Gebrauch mit 1 000 Gew.-Teilen des im Beispiel 1 verwendeten üblichen Futtermittels verdünnt.

**Patentansprüche** (für die Vertragsstaaten BE, CH, DE, FR, GB, IT, LI, NL, SE)

1. Arzneimittel und/oder Futtermittelkonzentrate mit antibakterieller Wirkung mit einem Gehalt an

   a) 2-[(Methoxycarbonylhydrazono)-methylen]-chinoxalin-1,4-dioxyd, *dadurch gekennzeichnet*, daß sie als weitere[n] Wirkstoff(e)
   b) 2-(Methyl)-5,7-di-(chlor)-8-(hydroxy)-chinolin enthalten.

2. Arzneimittel und/oder Futtermittelkonzentrate nach Anspruch 1, *dadurch gekennzeichnet*, daß sie zusätzlich

   c) 4-Dimethylamino-3,5,6,10,12,12a-hexahydroxy-6-methyl-1,11-dioxo-1,4,4a,5,5a,6,11,12a-octahydronaphthacen-2-carbonsäureamid enthalten.

3. Arzneimittel und/oder Futtermittelkonzentrate nach den Ansprüchen 1 bis 2, *dadurch gekennzeichnet*, daß sie zusätzlich

   d) 2,4-Diamino-5 [3',4',5'-tri-(methoxy)-benzyl]-pyrimidin enthalten.

4. Arzneimittel und/oder Futtermittelkonzentrate nach den Ansprüchen 1 bis 3, *dadurch gekennzeichnet*, daß sie 1 oder mehr in Arzneimitteln und/oder Futtermittelkonzentraten übliche[s] feste[s] und/oder flüssige[s] Konfektionierungsmittel enthalten.

5. Arzneimittel und/oder Futtermittelkonzentrate nach den Ansprüchen 1 bis 4, *dadurch gekennzeichnet*, daß sie zusätzlich 1 oder mehr Salicylsäurederivat(e) enthalten.

6. Arzneimittel und/oder Futtermittelkonzentrate nach den Ansprüchen 1 bis 5, *dadurch gekennzeichnet*, daß sie in räumlich getrennter Aufteilung der Wirkstoffe in ein und derselben Verpackungs- bzw. Applikationseinheit vorliegen.

7. Arzneimittel und/oder Futtermittelkonzentrate nach den Ansprüchen 1 bis 6, *dadurch gekennzeichnet*, daß das Gewichtsverhältnis von a) : b) : sofern vorhanden c) : sofern vorhanden d) 1 bis 5 : 0,5 bis 100 : 0,1 bis 100 : 0,6 bis 100 ist.

8. Verfahren zur Herstellung der Arzneimittel und/oder Futtermittelkonzentrate nach den Ansprüchen 1 bis 7, *dadurch gekennzeichnet*, daß man die Wirkstoffe und, falls verwendet, die Konfektionierungsmittel, miteinander vermischt.

**Patentansprüche** (für Vertragsstaat AT)

1. Verfahren zur Herstellung von Arzneimitteln und/oder Futtermittelkonzentraten mit antibakterieller Wirkung mit einem Gehalt an
   a) 2-[(Methoxycarbonylhydrazono)-methylen]-chinoxalin1,4-dioxyd und

   b) 2-(Methyl)-5,7-di-(chlor)-8-(hydroxy)-chinolin

   als weiteren Wirkstoff, *dadurch gekennzeichnet*, daß sie durch Vermischen dieser Wirkstoffe sowie gegebenenfalls von 1 oder mehreren in Arzneimitteln und/oder Futtermittelkonzentraten üblichen festen und/oder flüssigen Konfektionierungsmittel(n) hergestellt werden.

2. Verfahren nach Anspruch 1, *dadurch gekennzeichnet*, daß zusätzlich

c) 4-Dimethylamino-3,5,6,10,12,12a-hexahydroxy-6-methyl-1,11-dioxo-1,4,4a,5,5a,6,11,12a-octahydronaphtacen-2-carbonsäureamid zugemischt wird.

3. Verfahren nach den Ansprüchen 1 bis 2, *dadurch gekennzeichnet,* daß zusätzlich

d) 2,4-Diamino-5 [3',4',5'-tri-(methoxy)-benzyl]-pyrimidin zugemischt wird.

4. Verfahren nach den Ansprüchen 1 bis 3, *dadurch gekennzeichnet,* daß zusätzlich 1 oder mehr Salicylsäurederivat(e) zugemischt wird (werden).

5. Verfahren nach den Ansprüchen 1 bis 4, *dadurch gekennzeichnet,* daß die Wirkstoffe in räumlich getrennter Aufteilung in ein und derselben Verpackungs- bzw. Applikationseinheit konfektioniert werden.

6. Verfahren nach den Ansprüchen 1 bis 5, *dadurch gekennzeichnet,* daß ein Gewichtsverhältnis von a) : b) : sofern vorhanden c) : sofern vorhanden d) 1 bis 5 : 0,5 bis 100 : 0,1 bis 100 : 0,6 bis 100 eingehalten wird.


**Claims for the Contacting States: BE, CH, DE, FR, GB, IT, LI, NL, SE**

1. Medicaments and/or feed concentrates having an antibacterial activity and a content of

a) 2-(methoxycarbonylhydrazono)-methylene]-quinoxaline-1,4-dioxide, *characterized* in that it contains

b) 2-(methyl)-5,7-di-(chloro)-8-(hydroxy)-quinoline as another (other) active ingredient(s).

2. Medicaments and/or feed concentrates according to claim 1, *characterized* in that they additionally contain

c) 4-dimethylamino-3,5,6,10,12,12a-hexahydroxy-6-methyl-1,11-dioxo-1,4,4a,5,5a,6,11,12a-octahydronaphthacene-2-carboxylic acid amide.

3. Medicaments and/or feed concentrates according to claims 1 to 2, *characterized* in that they additionally contain

d) 2,4-diamino-5-[3',4',5'-tri-(methoxy)-benzyl]-pyrimidine.

4. Medicaments and/or feed concentrates according to claims 1 to 3, *characterized* in that they contain one or more solid and/or liquid carrier(s) which is (are) conventional in medicaments and/or feed concentrates.

5. Medicaments and/or feed concentrates according to claims 1 to 4, *characterized* in that they additionally contain one or more salicylic acid derivative(s).

6. A medicament and/or feed concentrate according to claims 1 to 5, *characterized* in that they are contained in one and the same packaging unit and/or unit for administration with spatially separated active ingredients.

7. Medicaments and/or feed concentrates according to claims 1 to 6, *characterized* in that the weight ratio of a): b): any c): any d) is 1 to 5 : 0.5 to 100 : 0.1 to 100 / 0.6 to 100.

8. A process of producing the medicaments and/or feed concentrates according to claims 1 to 7, *characterized* in that the medicaments and any carriers are mixed with each other.


**Claims for the Contracting State: AT**

1. A process for preparing medicaments and/or feed concentrates having an antibacterial activity and a content of

a) 2-[(methoxycarbonylhydrazono)-methylene]-quinoxaline1,4-dioxide and

b) 2-(methyl)-5,7-di-(chloro)-8-(hydroxy)-quinoline

as additional ingredient, *characterized* in that they are prepared by mixing these active ingredients and optionally 1 or more solid and/or liquid carrier(s) which is (are) conventional in medicaments and/or feed concentrates.

2. A process according to claim 1, *characterized* in that additionally

c) 4-dimethylamino-3,5,6,10,12,12a-hexahydroxy-6-methyl-1,11-dioxo-1,4,4a,5,5a,6,11,12a-octahydronaphthacene-2-carboxylic acid amide is mixed thereto.

3. A process according to the claims 1 to 2, *characterized* in that additionally

d) 2,4-diamino-5-[3',4',5'-tri-(methoxy)-benzyl]-pyrimidine is mixed thereto.

4. A process according to the claims 1 to 3, *characterized* in that additionally 1 or more salicylic acid derivative(s) is (are) mixed thereto.

5. A process according to the claims 1 to 4, *characterized* in that the active ingredients are packed spatially separated in one and the same packaging unit and/or unit for administration.

6. A process according to the claims 1 to 5, *characterized* in that a weight ratio of a) : b) : any c) : any d) is 1 to 5 : 0,5 to 100 : 0,1 to 100 / 0,6 to 100 is used.

**Revendications** pour les l'Etats Contractants BE, CH, DE, FR, GB, IT, LI, NL, SE

1. Médicaments et/ou concentrés de nourriture pour bétail à effet antibactérien, renfermant

a) du 1,4-dioxyde de 2-[(méthoxycarbonylhydrazono)-méthylène]-quinoxaline, *caractérisé* en ce qu'ils renferment, comme autre(s) substance(s) active(s)

b) de la 2-(méthyl)-5,7-di(chloro)-8-(hydroxy)-quinoléine.

2. Médicaments et/ou concentrés de nourriture pour bétail selon la revendication 1, *caractérisés* en ce qu'ils renferment, en plus

c) de l'amide d'acide 4-diméthylamino-3,5,6,10,12,12a-hexahydroxy-6-méthyl-1,11-dioxo-1,4,4a,5,5a,6,11,12aocta-hydronaphtacène-2-carboxylique.

3. Médicaments et/ou concentrés de nourriture pour bétail selon les revendications 1 à 2, *caractérisés* en ce qu'ils renferment, en outre,

d) de la 2,4-diamino-5-[3',4',5'-tri-(méthoxy)-benzyl]-pyrimidine.

4. Médicaments et/ou concentrés de nourriture pour bétail selon les revendications 1 à 3, *caractérisés* en ce qu'ils renferment un ou plusieurs produit(s) de confection solide(s) et/ou liquide(s), usuel(s) dans les médicaments et/ou les concentrés de nourriture pour bétail.

5. Médicaments et/ou concentrés de nourriture pour bétail selon les revendications 1 à 4, *caractérisés* en ce qu'ils renferment en plus un ou plusieurs dérivé(s) de l'acide salicylique.

6. Médicaments et/ou concentrés de nourriture pour bétail selon les revendications 1 à 5, *caractérisés* en ce qu'ils se présentent sous la forme d'une répartition séparée spatialement, des substances actives dans une seule et même unité d'emballage ou d'application.

7. Médicaments et/ou concentrés de nourriture pour bétail selon les revendications 1 à 6, *caractérisés* en ce que la proportion en poids de a) : b) : c) dans la mesure de sa présence : d) dans la mesure de sa présence est de 1 à 5 : 0,5 à 100 : 0,1 à 100 : 0,6 à 100.

8. Procédé de préparation des médicaments et/ou concentrés de nourriture pour bétail selon les revendications 1 à 7, *caractérisé* en ce qu'on mélange mutuellement les substances actives et, si on en utilise, les agents de confection.

**Revendications** pour l'Etat Contractant AT

1. Procédé pour la préparation des médicaments et/ou concentrés de nourriture pour bétail à effet antibactérien, renfermant

a) du 1,4-dioxyde de 2-[(méthoxycarbonylhydrazono)méthylène]-quinoxaline, et

b) de la 2-(méthyl)-5,7-di(chloro)-8-(hydroxy)-quinoléine comme autre substance active, *caractérisé* en ce qu'on mélange mutuellement les substances actives et, si on en utilise, 1 ou plusieurs produit(s) de confection solide(s) et/ou liquide(s), usuel(s) dans les médicaments et/ou les concentrés de nourriture pour bétail.

2. Procédé selon la revendication 1, *caractérisé* en ce que, en plus

c) de l'amide d'acide 4-diméthylamino-3,5,6,10,12,12a-hexahydroxy-6-méthyl-1,11-dioxo-1,4,4a,5,5a,6,11,12aocta-hydronaphtacène-2-carboxylique est mélangé.

3. Procédé selon les revendications 1 à 2, *caractérisé* en ce que, en plus

d) de la 2,4-diamino-5-[3',4',5'-tri-(méthoxy)-benzyl]-pyrimidine est mélangé.

4. Procédé selon la revendication 1, *caractérisé* en ce que 1 ou plusieurs dérivé(s) de l'acide salicylique est (sont) mélangé(s).

5. Procédé selon les revendications 1 à 4, *caractérisé* en ce que les substances actives sont confectionnes sous la forme d'une répartition séparée spatialement, dans une seule et même unité d'emballage ou d'application.

6. Procédé selon les revendications 1 à 5, *caractérisé* en ce que est observée une proportion en poids de a) : b) : c) dans la mesure de sa présence : d) dans la mesure de sa présence de 1 à 5 : 0,5 à 100 : 0,1 à 100 : 0,6 à 100.